# EUROPÄISCHE PATENTSCHRIFT

(11) **EP 0 661 070 B1**
(45) Veröffentlichungstag und Bekanntmachung des Hinweises auf die Patenterteilung: **19.05.1999**
(21) Anmeldenummer: 94118118.2
(22) Anmeldetag: 17.11.1994
(51) Int. Cl.: A61M 15/00

(54) **Vorrichtung zum Inhalieren**
Inhalation device
Dispositif d'inhalation

(30) Priorität: 30.11.1993 DE 4340768
(43) Veröffentlichungstag der Anmeldung: 05.07.1995
(73) Patentinhaber: BAYER AG, 51368 Leverkusen (DE)
(72) Erfinder: Herold, Heiko Dipl.-Ing, D-41470 Neuss (DE); Wollenschläger,Axel Dr, D-51467 Bergisch Gladbach (DE); Hegazy, Ahmed Dr, D-51375 Leverkusen (DE); Herboth,Matthias Dr, D-51375 Leverkusen (DE); Diederich,Reiner Dipl-Ing, D-50389 Wesseling (DE); Kleissendorf,Roland Dipl.-Ing, D-51469 Bergisch-Gladbach (DE); von Schuckmann, Alfred, D-47627 Kevelaer (DE)

(56) Entgegenhaltungen:
- EP-A- 0 505 321
- WO-A-82/01470
- WO-A-92/00771
- WO-A-92/09322
- DE-A- 4 004 904
- GB-A- 2 165 159

## Beschreibung

Die Erfindung bezieht sich auf eine Vorrichtung zum Inhalieren eines pulverförmigen, pharmakologisch wirksamen Arzneistoffs, bestehend aus einem Vorratsbehälter für den Arzneistoff, einer handbetätigten Dosiereinrichtung zur Bereitstellung einer abgeteilten Inhalierstoffmenge, die von dem beim Inhalieren angesaugten Luftstrom erfaßt und verwirbelt wird, einem Lufteinlaßkanal, einer Wirbelkammer und einem Verbindungskanal zwischen der Wirbelkammer und der Dosiereinrichtung sowie einem von der Wirbelkammer ausgehenden Mundstückkanal.

Eine Vorrichtung dieser Art ist durch die DE-OS 40 04 904 bekannt. Der Arzneistoff-Vorrat wird dort an der Peripherie der Dosiertrommel bereitgehalten und radial nachgestellt. Das Innere der Dosiertrommel enthält eine Steuervorrichtung zum aktiven Ausbringen der abgeteilten Arzneistoff-Inhaliermenge aus radial auswärts gerichteten, offenen Dosierausnehmungen. Dazu werden den Boden der winkelgleich verteilt angeordneten Dosierausnehmungen bildende Schieber zentral kurvengesteuert. Der diesbezügliche Aufwand ist erheblich, zumal die Steuermechanik von einer eine Längsseite der Vorrichtung nahezu voll einnehmenden Betätigungstaste ausgelöst wird. Lufteinlaßkanal und Verbindungskanal verlaufen stumpfwinklig tangierend zur Dosiertrommel, wobei sich dieser Luftführungstrakt über eine als Nebenhöhle gestaltete Wirbelkammer im wesentlichen gestreckt bis zum anderen Ende der Vorrichtung fortsetzt. Die Arzneistoff-Übergabestelle liegt nahe dem offenen Einsaugende und der Stoff kann dort leicht ungenutzt herausfallen. Außerdem sind die dosierten Arzneistoff-Inhaliermengen durch die Zwangsleerung der Dosierausnehmung addierbar; es kann daher zu einer gefährlichen Überdosierung kommen. Für die Vorratshaltung verbleibt volumenmäßig nur ein Bruchteil der im Taschenformat ausgebildeten Vorrichtung. Die Handhabungsweise ist nicht eindeutig.

Aufgabe der Erfindung ist es, eine gattungsgemäße Vorrichtung unter baulicher Vereinfachung gebrauchsvorteilhafter und gebrauchssicherer auszubilden, insbesondere auch strömungstechnisch zu verbessern sowie durch vollen Austrag der abgeteilten Arzneistoff-Inhaliermenge die Wirkung gleichzuhalten.

Gelöst ist diese Aufgabe durch die im Anspruch 1 angegebene Erfindung.

Die daran anschließenden Ansprüche geben weitere vorteilhafte Ausgestaltungen der erfindungsgemäßen Vorrichtung wieder.

Zufolge solcher Ausgestaltung ist eine gattungsgemäße Vorrichtung vereinfachten Aufbaus und erhöhten Gebrauchs- und Sicherheitswerts erzielt. Der mechanische Aufwand ist stark reduziert. Es bedarf keiner koordinierten Drucktastenbewegung und Einatmung. Die Menge wird in Ruhe in die Ausgabebereitschaftsstellung gebracht. Der Benutzer kann sich auf das Einatmen des Wirkstoffes konzentrieren. Konkret ist der Inhalator so ausgebildet, daß die Dosiereinrichtung aus einer Dosiertrommel besteht, bei der der Vorratsbehälter für den Arzneistoff im Innenraum der Dosiertromnmel angeordnet ist, und daß der Lufteinlaßkanal und der Verbindungskanal V-förmig aufeinander und auf eine durch die Dosiertrommel im Scheitel der beiden Luftkanäle bereitgestellte, in Form einer Kuppel vorliegende, abgeteilte Arzneistoff-Menge zulaufen. Der Innenraum der Dosiertrommel ist nun für die Vorratsbildung genutzt. Ihr Inhalt wird in Bewegung gehalten. Er gelangt auf kürzestem Wege in die Ausgabereitschaftsstellung. Dort erfolgt eine restfreie Austragung. Es können aufgrund der geschilderten Ausrichtung und Aneinanderreihung der Funktionsbereiche keine Partikel zurückfallen. Der Saugstrom trifft eingangs auf den V-Scheitel und strömt in die exakt positionierte und abgeteilte, exponierte Anhäufung des Arzneistoffes. Die kuppelförmig dargebotene Zone wird rasch abgetragen, verwirbelt bzw. dispergiert und bei der Einatmung an den Zielort verbracht. Der Arzneistoff wird gegen die Wirkung der Schwerkraft gesaugt. Fehlhaltungen sind praktisch ausgeschlossen, da die Lage der Dosiertrommel die korrekte Handhabung suggeriert und der am anderen, oberen Ende liegende Mundstückkanal als Übergabebrücke dem Benutzer unschwer auffällt. Außerdem kann sich kein Potential für eine Überdosierung bilden; eine nicht inhalierte Inhaliermenge verschwindet in Richtung Vorrat. Sie wird nicht durch den nächsten Schaltschritt ergänzt. Als strömungstechnisch vorteilhaft erweist sich sodann die Maßnahme, daß der Verbindungskanal radial zur Dosiertrommel verläuft. Er wird so seiner Funktion als Beschleunigungskanal mit nach oben hin zunehmender Beruhigung bestens gerecht. Um einen genügend großen Übergangsbereich zur Wirbelkammer, welche zugangsseitig als Verweilzeitkammer fungiert, zu schaffen, ist der Inhalator vorteilhaft so ausgebildet, daß der Verbindungskanal sich zur Wirbelkammer hin verbreitert und die Spurgerade der einen Wand im wesentlichen radial zum Zentrum der Wirbelkammer liegt und diejenige der anderen Wand im wesentlichen tangential dazu verläuft. Dies führt zu einer verwirbelungsgünstigen, rotierrichtungsbestimmenden Strömung. Im Hinblick auf den abführenden Mundstückkanal ist die Konstruktion dabei so gewählt, daß der Mundstückkanal stumpfwinkling zum Verbindungskanal verläuft und die Spurgerade der einen Wand etwa tangential und diejenige der anderen Wand etwa radial zum Zentrum der Wirbelkammer ausgerichtet sind. Dabei wird zweckmäßig auch von einer kontinuierlichen Querschnittsverbreitung (auch in die Querbreite gehend) Gebrauch gemacht (Verwirklichung der erstrebten Diffusor-Wirkung). Was nun die Ausbildung des Lufteinlaßkanals betrifft, so ist diesbezüglich so vorgegangen, daß die Spurgerade der Innenwand und die Spurgerade der Außenwand des Lufteinlaßkanals sekantenförmig zur Dosierkammer ausgerichtet sind. Ansaugöffnung und Luftaustrittsöffnung der Vorrichtung sind dadurch räumlich ausreichend beabstandet. Was nun die Ausbildung der Dosiertrommel betrifft, so ist diese in der Weise weitergeführt, daß sie eine mit Abteildurchbrüchen ausgestattete Drehhülse besitzt. Die in gleicher Winkelverteilung angeordneten Abteildurchbrüche tauchen aufgrund der mischtrommelartigen Drehung der Dosiertrommel in den Pulvervorrat ein, ohne daß es zu Pressungen etc. kommt. Die Dosierung ist einschließlich der vorletzten Abteilung und gegebenenfalls bis zur letzten Abteilung volumengerecht. Es kommt auch nicht zu einer Blockbildung des Arzneistoffes aufgrund der Nutzung der Trommelbewegung. Weiter wird vorgeschlagen, daß die Drehhülse von der Mantelwand eines Topfes gebildet ist, dessen Boden eine Drehhandhabung bildet. Die entsprechend nach außen hin zugängliche Drehhandhabe ist äußerst bedienungseinfach und direkt. Um zu einer definierten Lage der Kuppel, also der dosierten Menge des abgeteilten Arzneistoffes, zu gelangen, ist ein den Drehsinn bestimmendes Rastgesperre zwischen Drehhülse und Gehäuse der Vorrichtung zugeordnet. In bezug auf die Drehlagerung der Dosiertrommel und die Erzielung stets gleicher Arzneistoff-Inhaliermengen erweist es sich als vorteilhaft, daß die von einer Außenwand umfaßte Drehhülse über einen Teilwinkelbereich von einem inneren Wandabschnitt unterfangen ist, welcher im Bereich des V-Scheitels den Boden der von jeweils einer der Durchbrechungen gebildeten Abteilkammer bildet. In diesem Zusammenhang ist es zudem vorteilhaft, daß die Querwände der Durchbrechungen zum Zentrum der Dosiertrommel hin keilförmig verlaufen. Dies führt bezüglich der Querwände zu einem sich V-scheitelseitig, also nach oben gerichtet, weitenden, konvex gebogenen, trapezförmigen Trog als Abteilkammer. Eine Weiterbildung der Erfindung besteht darin, daß ausgehend vom Boden des Topfes und/oder dem Gehäuse Auflockerungsfinger in die Dosiertrommel vorstehen. Sie bilden eine Art Rührwerk, das - aktiv oder statisch wirkend - die Auflockerung des pulverförmigen Arzneistoffes (Formulierung) optimiert. Um die nicht ganz zu verhindernde Feuchte zu eliminieren, ist dem Boden des Topfes oder der Innenseite eines Gehäuse-Fülldeckels eine Trockenmittelkammer zugeordnet, die entsprechend zu bestücken wäre. Ein solcher Fülldeckel kann zugleich als Sichtglas realisiert sein. Eine gute Einsaugwirkung wird erreicht, wenn am Einsaugende des Lufteinlaßkanals quergerichtete Lufteinlaßschlitze angebracht sind. So wird vermieden, daß sich die Ansaugöffnung zusetzt, beispielsweise durch lose mitgeführte Münzen etc.. Die gitterförmige Ausbildung des Lufteinlasses ist auch insofern nützlich, als keine Fremdpartikel wie Staubflusen etc. eingesogen werden können. Solche Lufteinlaßschlitze sind zweckmäßig auf beiden Breitseiten des Vorrichtungs-Gehäuses vorgesehen. Sie liegen so, daß sie bei der Handhabung des Inhalators nicht ohne weiteres zugehalten werden können; in jedem Falle bleibt die eine oder andere Seite offen. Weiter ist die Vorrichtung gekennzeichnet durch einen Teilungszwickel für den Weg der eingesaugten Luft. So entsteht ansaugseitig praktisch ein Y-Kanal. Um bei fehlerhafter Benutzung (statt zu saugen, zu blasen) den Austrag des pulverförmigen Arzneistoffes zu vermeiden, sind den Lufteinlaßschlitzen Exhalations-Sperrzungen zugeordnet. Durch Pusten gelangt so keine Atemluftfeuchtigkeit in das möglichst einwandfrei trockenzuhaltende Innere des Inhaliergeräts. Es kann sich hier um einfache Ventillappen handeln. Zu deren Montage kann dabei der oben erwähnte Teilungszwickel herangezogen werden, wie das der spezielle Teil der Beschreibung ausführt. Der gebrauchsgerechten Handhabung förderlich ist sodann die Maßnahme, daß die Lufteinlaßschlitze etwa bis auf die Höhe des Zentrums der Wirbelkammer reichen. Sie sind also aus dem Basisbereich genügend hochverlegt und können durch die Hand des Benutzers praktisch nicht verschlossen werden. Eine ebenfalls im Sinne der korrekten Handhabung nützliche Ausgestaltung wird durch eine etwa unterhalb der Wirbelkammer liegende Fingermulde des Vorrichtungsgehäuses erreicht. Die Fingermulde schafft zugleich eine Nische für die Unterlippe des Benutzers, ohne daß das Mundstück eine den Gesamtquerschnitt des Gehäuses überschreitende Auskragung haben müßte. Es liegt eine benutzungsvorteilhafte Schnabelstruktur vor. Dabei läßt sich das Ganze durch eine das Gehäuse überfangende Schutzkappe abdecken. Letztere kann durch Klemmung, Rastung etc. am Gehäuse in einfacher Weise gehalten sein, so daß die Vorrichtung nur durch willensbetonten Zug an der Schutzkappe freigegeben werden kann. Insoweit ist auch diesbezüglichen hygienischen Erfordernissen Rechnung getragen. Um auch im Mundstückbereich anstehende Feuchte in der Zeit des Nichtgebrauchs zu eliminieren, ist auch der Schutzkappe eine Trockenmittelkammer zugeordnet. Eine weitere vor allem herstellungstechnisch günstige Verbesserung besteht darin, daß alle Luftkanäle und die Wirbelkammer in einem von zwei Gehäuseschalen umschließbaren Träger eingearbeitet sind, in dem auch die Dosiertrommel gelagert ist, wobei die Gehäuseschalen die Abdeckung für die Luftkanäle und die Wirbelkammer bilden. Außerdem kann vorgesehen werden, daß der Träger den Standfuß der als Standgerät gestalteten Vorrichtung formt, dessen Schulter einen Aufsteckbegrenzungsanschlag für die Schutzkappe bildet. Endlich besteht noch eine strömungstechnisch interessante Einzelheit der Kanalstruktur darin, daß der dem oberen Ende des Verbindungskanals zugewandte Wandabschnitt der Wirbelkammer eine geringere Wölbung besitzt und damit einen deutlich flacheren Verlauf einnimmt, zu welchem Wandungsabschnitt ein zentraler Durchlaß der Wirbelkammer exzentrisch angeordnet ist. Diese "Verflachung" des oberen Halbkreises führt zu dem überraschenden Effekt, daß eine dort im allgemeinen zu beobachtende Neigung zur Ablagerung essentiell reduziert ist bzw. praktisch nicht mehr auftritt. Anstelle eines reinen kreisförmigen Strömungsweges tritt ein relativ ansteigender Strömungsweg mit einer stärkeren Strömungswirkung, der die zur Anhaftung neigenden Partikel sicher mitreißt.

Mit der Erfindung werden folgende weitere Vorteile erzielt:
- Der Inhalator eignet sich auch für Arzneistoff-Formulierungen, die schlecht fließfähig sind oder zum Anbacken neigen. Aufgrund der gleichzeitigen Homogenisierung bei jedem Dosiervorgang werden evtl. vorhandene Pulverbrücken aufgebrochen und aufgelockert.
- Ferner hat sich herausgestellt, daß sich bei geringen Abweichungen von der vorgeschriebenen Gebrauchslage die Dosiergenauigkeit nicht merklich verschlechtert; d.h. geringe Abweichungen von der Gebrauchslage sind im Hinblick auf die Dosiergenauigkeit unkritisch. Nach der Dosierung d.h. nach Betätigung der Dosiertrommel, kann die Inhalation in jeder beliebigen Lage erfolgen.
- Aufgrund der spritzgußfreundlichen Konstruktion und aufgrund der geringen Zahl von erforderlichen Bauteilen kann der Inhalator kostengünstig und wirtschaftlich in großen Stückzahlen hergestellt werden.
- Die besondere Strömungsführung im Anschluß an die Austragung aus einer Abteilkammer in der Dosiertrommel führt dazu, daß ein hoher Anteil von lungengängigen (respirablen) Pulverpartikeln erzeugt wird.

Der Gegenstand der Erfindung ist nachstehend anhand eines zeichnerisch veranschaulichten Ausführungsbeispieles näher erläutert. Es zeigen:
- Fig. 1: die Vorrichtung zum Inhalieren eines pulverförmigen Arzneistoffes, und zwar in partiellem Vertikalschnitt gemäß Linie I-I in Fig. 2 und bei vollständig geschnittener Schutzkappe,
- Fig. 2: den Schnitt gemäß Linie II-II in Fig. 1,
- Fig. 3: einen die Kanalstruktur etc. mitbildenden Träger des Gehäuses in isolierter Darstellung in Seitenansicht,
- Fig. 4: den Schnitt gemäß Linie IV-IV in Fig. 3,
- Fig. 5: den Schnitt gemäß Linie V-V in Fig. 3, den den Ansaugweg spaltenden Teilungszwickel nebst Exhalations-Sperre wiedergebend,
- Fig. 6: den Träger in Einzeldarstellung, von der anderen Breitseite gesehen,
- Fig. 7: den Schnitt gemäß Linie VII-VII in Fig. 6,
- Fig. 8: den Schnitt gemäß Linie VIII-VIII in Fig. 6, die Aufnahme für das Rastgesperre wiedergebend,
- Fig. 9: eine Innenansicht gegen die rechtsseitige Schale des Gehäuses,
- Fig. 10: den Schnitt gemaß Linie X-X in Fig. 9,
- Fig. 11: den Schnitt gemäß Linie XI-XI in Fig. 9,
- Fig. 12: eine Innenansicht gegen die linksseitige Schale des Gehäuses,
- Fig. 13: den Schnitt gemaß Linie XIII-XIII in Fig. 12,
- Fig. 14: den Schnitt gemäß Linie XIV-XIV in Fig. 12,
- Fig. 15: die Dosiertrommel in Einzeldarstellung, in die Topfhöhlung gesehen,
- Fig. 16: den Schnitt gemäß Linie XVI-XVI in Fig. 15,
- Fig. 17: die Dosiertrommel in Einzeldarstellung, gegen ihre Drehhandhabe gesehen,
- Fig. 18: das dosiertrommelseitige Detail des Rastgesperres,
- Fig. 19: eine Herausvergrößerung des die Abteilkammer bildenden V-Scheitel-Bereichs und
- Fig. 20: den Schnitt gemaß Linie XX-XX in Fig. 19.

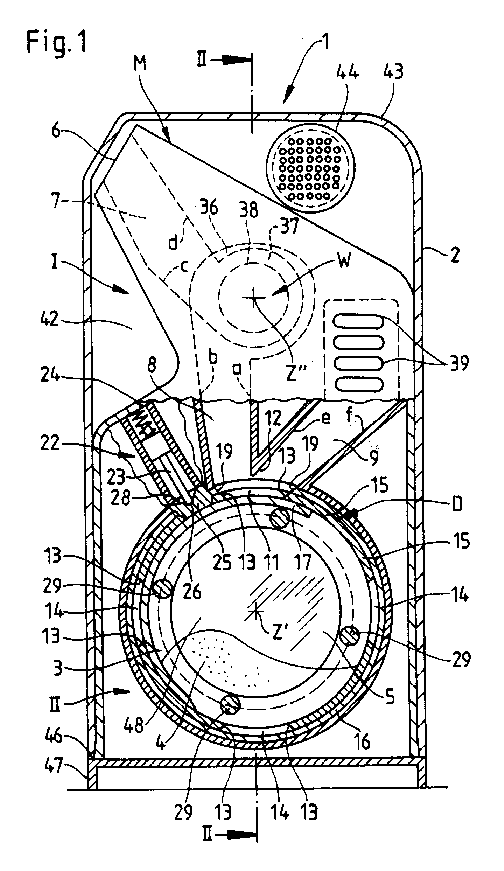

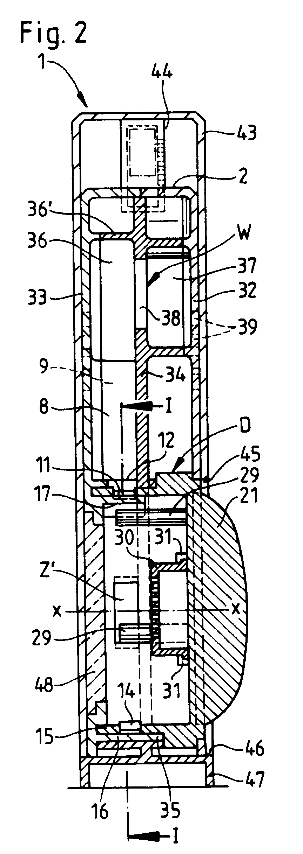

Die als Taschengerät ausgebildete Vorrichtung, nachstehend Inhalator 1 genannt, besitzt gemäß Fig. 1 und 2 ein langrechteckiges, im Taschenformat ausgebildetes, flaches Gehäuse 2. Dessen Kopfbereich enthält einen sogenannten Dispergierteil I und dessen Fußbereich einen Dosierteil II. Bei der nachfolgenden Beschreibung wird vorausgesetzt, daß sich der Inhalator 1 in einer Vertikalebene befindet, die in den Figuren mit der Zeichenebene übereinstimmt; d.h. wie in den Fig. 1 und 2 dargestellt, ist der Dispergierteil I oberhalb des Dosierteils II angeordnet.

Das Dosierteil II enthält einen Vorratsbehälter 3, der mit pulverförmigem Arzneistoff 4 (Formulierung) gefüllt ist.

Der Vorratsbehälter 3 ist vom Innenraum 5 einer Dosiertrommel D gebildet. Die Dosiertrommel D ist drehbar im Gehäuse 2 gelagert und von außen her unmittelbar handbetätigbar. Sie befindet sich im Fußbereich des Inhalators 1 und nutzt volumenmäßig nahezu die gesamte Breite des Gehäuses 2. Die geometrische, horizontal liegende Drehachse der Dosiertrommel D ist mit x-x bezeichnet (Fig. 2).

Oberhalb der zur Bereitstellung einer abgeteilten Arzneistoff-Inhaliermenge dienenden Dosiertrommel D befindet sich eine Wirbelkammer W.

Die Wirbelkammer setzt sich in ein nach oben gerichtetes Mundstück M fort, welches an höchster Stelle seines schnabelförmigen Gebildes eine mundgerechte Austrittsöffnung 6 formt.

Der Inhalator wird beim Gebrauch so gehalten, daß sich das Dosierteil II unten und das Dispergierteil I mit dem Mundstück M oben befindet (Gebrauchsstellung). Diese Stellung entspricht auch der in Fig. 1 und Fig. 2 gewählten Darstellung.

Dosiertrommel D, Wirbelkammer W und Mundstückkanal 7, welcher die Wirbelkammer W und die Austrittsöffnung 6 miteinander verbindet und mit seinem Mantel das Mundstuck M bildet, liegen also in der Gebrauchsstellung des Inhalators 1 praktisch vertikal übereinander. Die pulverförmigen Partikel werden somit bei der Inhalation von unten nach oben gefördert.

Von der Wirbelkammer W geht ein nach unten gerichteter Verbindungskanal 8 aus. Letzterer schließt im Bereich der Bereitstellung einer vorgegebenen (abgeteilten) Arzneistoff-Inhaliermenge an einen nach außen führenden Lufteinlaßkanal 9 an.

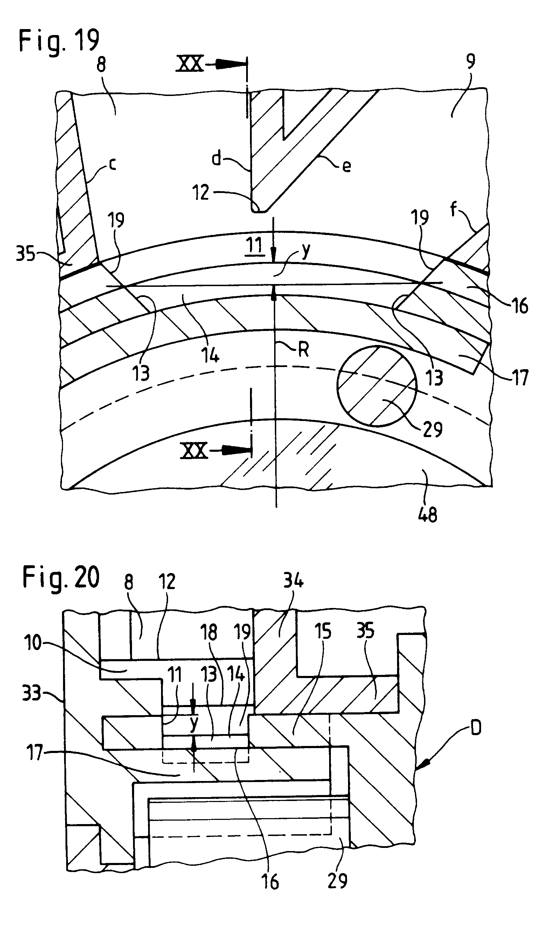

Lufteinlaßkanal 9 und Verbindungskanal 8 verlaufen V-förmig zueinander und auf die Peripherie der Dosiertrommel D zu. Es handelt sich um eine spitzwinklige Kanalstruktur (ca. 50°), in deren V-förmigem Scheitelbereich die exakt dosierte, aus dem Vorratsbehälter 3 entnommene Pulvermenge dem Saugluftstrom zur Austragung dargeboten wird. Die Dosiertrommel D weist zu diesem Zweck kanalseitig offene Abteilkammern 11 auf. Aufgrund der kreisförmigen Wölbung der Dosiertrommel D wird der abgeteilte Arzneistoff (Formulierung) in Gegenrichtung zum Kehrenverlauf als vorgewölbte Schicht bereitgestellt. Die daraus resultierende Arzneistoff-Kuppel läßt sich besonders strömungsgünstig ab-bzw. ausräumen. Die in Richtung einer Kanalscheide 12 der Kanäle 8 und 9 gehende, konvexe Wölbung geht besonders deutlich aus Fig. 19 hervor. Dort ist die aus der Wölbung der Kammer 11 resultierende Überhöhung y (angegeben in der Sehnenmitte) der auf das Zentrum Z' der Dosiertrommel D gerichteten Radialen R gut erkennbar. Das Zentrum Z' liegt auf der erwähnten geometrischen Drehachse x-x.

Wie aus den Fig. 1, 2, 12 bis 14, 16 und insbesondere 19 ersichtlich ist, wird eine Abteilkammer 11 durch einen Durchbruch 14 in einer Drehhülse 15 der als Topf gestalteten Dosiertrommel D gebildet. Gemäß Fig. 1 sind vier Durchbrüche 14 gleichmäßig über den Umfang der Drehhülse 15 verteilt. Die Durchbrüche 14 sind durch Seitenwände 13 begrenzt, die derart abgeschrägt sind, daß sich die Durchbrüche 14 zum Zentrum Z' der Dosiertrommel D hin verjüngen. Der Schrägungswinkel liegt bei 45° zur kürzesten Verbindung zwischen Außen- und Innenfläche der Dosiertrommel-Wandung. Die Querwände 13 verleihen der Abteilkammer 11 die Form eines trapezförmigen, nach außen öffnenden, gebogenen Troges von deutlich größerer Länge als Breite.

Die Drehhülse 15 ist drehbar zwischen einer Ringwand 16 und einem ebenfalls ringförmigen, sich über einen Bogenwinkel von ca. 180° erstreckenden Wandungsabschnitt 17 gelagert. Der etwa halbkreisförmige, stegförmige Wandungsabschnitt 17 ist dabei so angeordnet, daß er einerseits am Zenit der Dosiertrommel D über den V-förmigen Scheitelbereich der Luftkanäle 8 und 9 so weit hinausragt, daß zu keinem Zeitpunkt eine durchgehende Verbindung zwischen den Luftkanälen 8 und 9 und dem Vorratsbehälter 3 besteht und andererseits am unteren Ende die Fußzone der Drehhülse 15 freibleibt (s. Fig. 1). Die Ringwand 16 und der Wandungsabschnitt 17 sind an dem Gehäuse 2 angeformt. Die Lagerung der Drehhülse kann durch einen etwa 1 bis 2 mm hohen Lagerkragen verbessert werden, der sich in Verlängerung des besagten Wandungsabschnitt 17 über den verbleibenden Umfangswinkel erstreckt und ebenfalls am Gehäuse 2 angeformt ist. Auf diese Weise wird die Mantelfläche der Drehhülse 15 in dem Ringspalt zwischen der Ringwand 16 und dem Wandungsabschnitt 17 einschließlich des erwähnten Lagerkragens exakt drehbar geführt. Die Ringwand 16 weist gegenüber der Kanalscheide 12 der Luftkanäle 8 und 9 ein Eintrittsfenster 18 auf. Die Seitenflächen 19 des Eintrittsfensters 18 sind in der Weise abgeschrägt, daß sie mit den Seitenflächen 13 der Abteilkammer 11 fluchten, wenn sich die Abteilkammer 11 genau unter dem Eintrittsfenster 18 befindet. Solange sich eine Abteilkammer 11 innerhalb des Winkelbereichs des Wandungsabschnitts 17 befindet, fungiert die Außenfläche des Wandungsabschnitts 17 als Boden für die trogförmige Abteilkammer 11. Aus diesem Grund ist die Höhe des stegförmigen Wandungsabschnitts 17 etwa größer bemessen als die Breite des Durchbruchs 14. Bei einer weiteren Drehung der Drehhülse 15 im Uhrzeigersinn, d.h. nachdem sich die Abteilkammer 11 unter dem Eintrittfenster 18 hinweg bis über das rechte obere Ende des Wandungsabschnitts 17 hinausgeschoben hat, ist der Boden der Abteilkammer 11 offen, so daß sie wieder mit dem Innenraum 5 bzw. mit dem Vorratsbehälter 3 in Verbindung steht.

Die etwa auf die Quermitte der Abteilkammer 11 weisende Kanalscheide 12 liegt in einem Abstand zum darunterliegenden Wandungsabschnitt 17, der etwa die Hälfte der Querabmessung des V-scheitelseitigen Endes des Verbindungskanals 8 bzw. des Lufteinlaßkanals 9 beträgt. Daraus resultiert eine hohe Strömungsgeschwindigkeit im Bereich der Kehre, so daß die mit dem pulverförmigen Arzneistoff gefüllte Abteilkammer 11 beim Inhalieren auch in einer kritischen Lage des Inhalators restfrei entleert wird.

Während der besagte Wandungsabschnitt 17 im Bereich des V-Scheitels mit seiner Außenfläche als Boden fungiert, deckt er im Bereich der ansteigenden, im Uhrzeigersinn vorgelagerten Abteilkammer die aus dem Pulvervorrat genommene Inhaliermenge des nächstfolgenden Durchbruchs 14 von innen her gegen den Vorrat ab. In Fig. 1 befindet sich diese Abteilkammer gerade in einer "9 Uhr-Stellung", während die zum Inhalieren bereitstehende Abteilkammer in einer "12 Uhr-Position" steht. Das Laden einer Abteilkammer 11 erfolgt in der Weise, daß sich der dazugehörige Durchbruch 14 beim Weiterdrehen der Drehhülse 15 unter dem Pulvervorrat 4 hindurchschiebt (in Fig. 1 etwa in einer Zone von "16 Uhr" bis "19 Uhr"), wobei sich diese Abteilkammer 11 mit dem Pulver füllt. Bei der weiteren Drehung der Drehhülse 15 gelangt diese Abteilkammer in den ansteigenden Bereich zwischen "18 Uhr" und "21 Uhr". In diesem Bereich erfolgt die Mengensicherung der Inhalationsdosis in der Abteilkammer durch den absperrend wirkenden Wandungsabschnitt 17, dessen unteres Ende als Abstreifer fungiert, so daß ein eventuell vorhandener einwärts gerichteter Pulverüberstand abgeschabt wird. Auf diese Weise wird eine genaue Inhalationsdosis mit reproduzierbarer Pulverdichte erreicht.

Der Wandungsabschnitt 17 läßt gut 180° zum Innenraum 5 des Vorratsbehälters 3 hin offen. Die Drehhülse 15 wirkt wie ein Drehschieber. Wird die dosierte Inhaliermenge nicht entnommen, gelangt sie beim Weiterdrehen der Dosiertrommel D automatisch wieder in den Vorrat, also den Vorratsbehälter 3, zurück. Eine Mengenaddition ist nicht möglich. Dieser wesentliche Vorteil ergibt sich aus der Vertikalorientierung der Funktionsorgane.

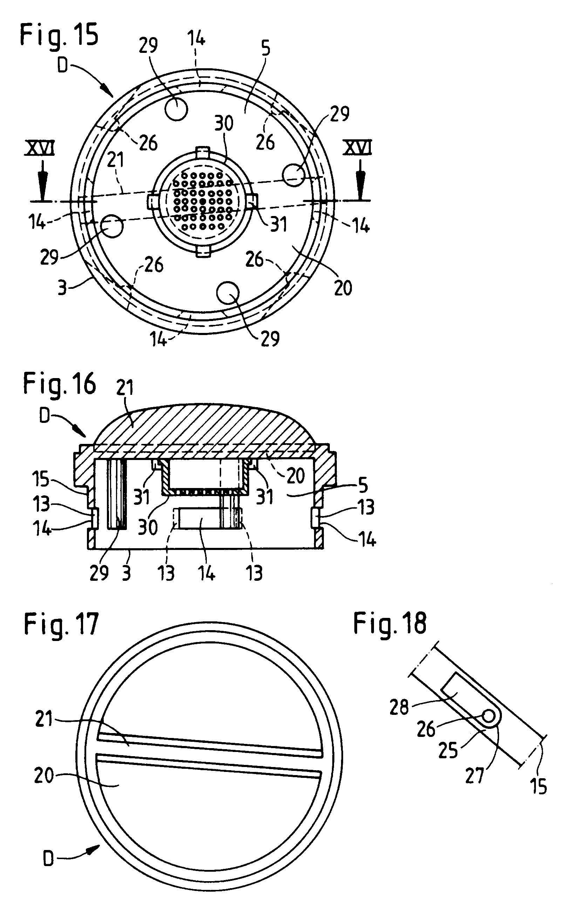

Die Mantelwandung der Drehhülse 15 der als Topf gestalteten Dosiertrommel D geht nach außen hin in einen den Vorratsbehälter 3 abschließenden Topfboden 20 über, an dem außenseitig ein bequem zugänglicher Drehknopf 21 angeordnet ist (siehe insbesondere Fig. 16 und Fig. 17).

Die Dosiertrommel D besitzt ein auf eine Drehung im Uhrzeigersinn ausgelegtes Rastgesperre 22. Hauptbestandteil desselben ist ein radial beweglich angeordneter Raststift 23, der in Eingriffsrichtung unter Belastung einer Druckfeder 24 steht. Das Gegenrastmittel sind am Umfang der Drehhülse 15 respektive der Dosiertrommel D liegende Gegenrasten 25, die so positioniert sind, daß Schritt für Schritt genau die Ausgabebereitschaftsstellung der Abteilkammer 11 erreicht wird. Die Gegenraste 25 ist flankenmäßig so ausgestattet, daß unter willensbetonter Überwindung die in der jeweiligen Ausgabebereitschaftsstellung eingerastete Dosiertrommel D weitergedreht werden kann, während eine Drehung in Gegenrichtung aufgrund einer steileren Flanke nicht ohne weiteres möglich ist. Eine Umkehrung des Drehsinns ist natürlich möglich, wenn das Gerät speziell für Linkshänder ausgelegt werden soll.

Die eigentliche Rastmulde der Gegenraste 25 ist in Fig. 18 mit 26 bezeichnet, die steilere Flanke mit 27 und die willensbetont überwindbare flachere Flanke mit 28. Die Gegenraste 25 liegt in einem wandungsverdickten, dem Topfboden 20 der Dosiertrommel D naherliegenden Bundabschnitt der Drehhülse 15.

Am Topfboden 20 sind parallel zur Drehachse der Dosiertrommel gleichmäßig über den Umfang verteilt mehrere Auflockerungsfinger 29 angebracht, die in den Pulvervorrat hineinragen und deren radialer Abstand vom Zentrum so gewählt ist, daß sie bei der Drehung den Rücken des Wandungsabschnitt 17 abstreifen, so daß sich dort kein Pulver festsetzen kann. Im Uhrzeigersinn gesehen, ist gemäß Fig. 1 unmittelbar hinter jedem Durchbruch 14 (voreilend) ein Auflockerungsfinger 29 angeordnet. Alternativ oder auch zusätzlich können die Auflockerungsfinger ortsfest am Gehäuse 2 angebracht sein, wobei ebenfalls die Trommelbewegung zur Auflockerung des Pulvers genutzt wird.

Im Zentrum des Bodens 20, ebenfalls innenseitig desselben, also in den Vorratsbehälter 3 ragend, befindet sich eine Trockenmittelkammer 30. Es handelt sich z.B. um eine mittels Klip-Verschluß befestigbare Kapsel, deren Boden wasserdampfdurchlässig ist. Zur Halterung der Kapsel dienen randübergreifende Klipnasen 31.

Das Inhalatorgehäuse 2 besteht aus zwei im Querschnitt U-förmigen Gehäuseschalen 32 und 33. Die beiden Schalen 32 und 33 umschließen sandwich-artig einen Träger 34 als Kernteil, in dem als wesentliche Bestandteile der Verbindungskanal 8, der Lufteinlaßkanal 9 mit einlaßseitiger Strömungsaufteilung, die Wirbelkammer W, der Mundstückkanal 7, eine kreisrunde Aussparung 35 für die Halterung der Dosiertrommel D und die Rastsperre 22 untergebracht sind. Die Luftkanäle 7, 8, 9 sind in Form von rinnenartigen Vertiefungen mit annähernd rechteckigem Querschnitt und die Luftkammern in Form von im wesentlichen kreisrunden Aussparungen in den Träger 34 eingearbeitet und werden durch die Gehäuseschalen 32 und 33 abgeschlossen bzw. abgedeckt. Diese Konstruktion gewährleistet auch dann eine ausreichende mechanische Stabilität, wenn der Träger 34 aus Gründen der Gewichtsersparnis relativ dünnwandig (z.B. ca. 5 mm) gestaltet wird.

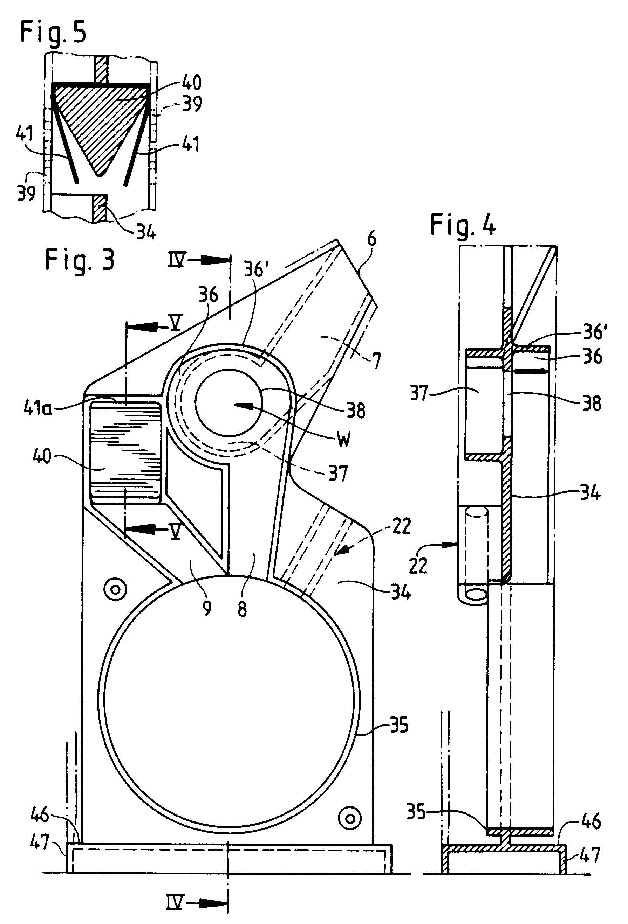

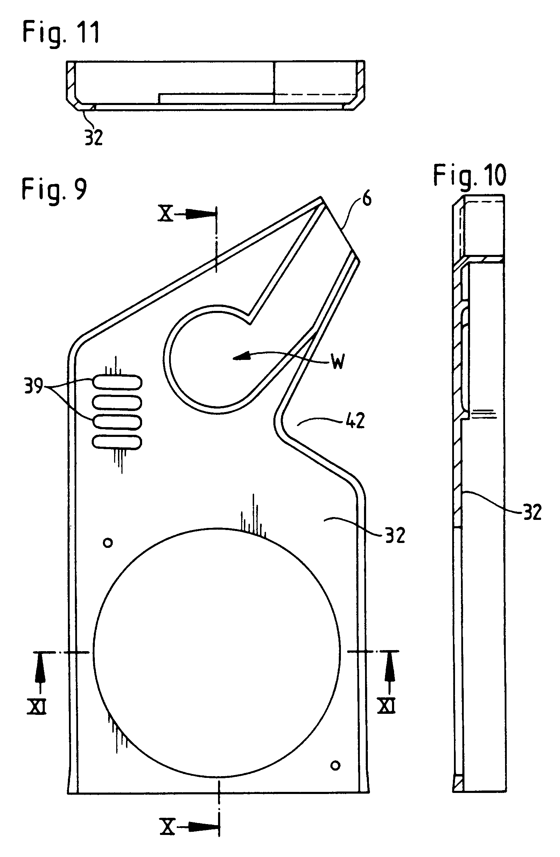

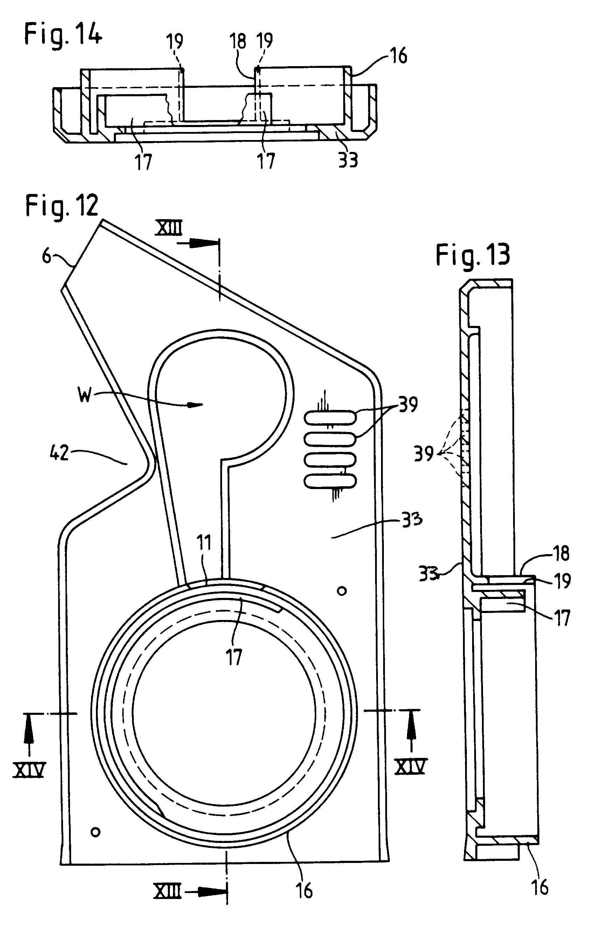

Der Träger 34 ist in den Fig. 3 bis 7 wiedergegeben, die gemäß Betrachtungsweise in Fig. 2 rechtsseitige Gehäuseschale 32 ergibt sich aus den Fig. 9 bis 11 in Einzeldarstellung und die linksseitige Gehäuseschale 33 aus den Fig. 12 bis 14. An den Innenseiten der Schale angeformte Leisten treten mit ihren Stirnenden gegeneinander und auch gegen entgegengerichtete Leisten des zwischengefaßten Trägers 34. Es kann eine klemmtechnische Befestigungsweise der Teile zugrundeliegen; eine Verklebung oder thermische Anbindung ist ebenfalls denkbar, wenn nicht zu Reinigungszwecken respektive Nachfüllzwecken die völlige bzw. teilweise Zerlegbarkeit des Gehäuses 2 angestrebt wird. In diesem Falle sind dann die üblichen klassischen Befestigungsmittel, wie Verschraubung etc., vorzuziehen.

Der Verbindungskanal 8 ist im Träger 34 so ausgerichtet, daß er im wesentlichen radial zur Dosiertrommel D verläuft. Während er sich in dieser Richtung leicht verjüngt, geht er in Gegenrichtung in eine kontinuierliche Verbreiterung über. In welchem Maße sich der Verbindungskanal 8 zur Wirbelkammer hin verbreitert, ergibt sich deutlich aus Fig. 1 und Fig. 3. Dort ist auch entnehmbar, daß die Spurgerade der einen Kanalwand a radial zum Zentrum Z' der kreisrunden Wirbelkammer W und die Spurgerade der anderen Wand b tangential zur Wirbelkammer verläuft. So liegt eine den Trillerpfeifen vergleichbare Umrißgestalt von Verbindungskanal 8 und Wirbelkammer W vor. Die Wirbelkammer ist zweiteilig ausgebildet, wobei die beiden Kammerteile, die Verweilzeitkammer 36 und die Auslaßkammer 37, senkrecht zu den Gehäuseschalen 32 und 33 gesehen, nebeneinander liegen und über einen zentralen, quergerichteten, kreisrunden Durchlaß 38 miteinander in Verbindung stehen. Durch diese Anordnung wird eine Speichelscheide geschaffen. Es wurde gefunden, daß Ablagerungen in der Verweilzeit 36 und Auslaßkammer 37 stark reduziert bzw. vermieden werden können, wenn das Kammervolumen durch eine nach innen gerichtete Mulde (Delle) in den Gehäuseschalen 32 und 33 verringert wird. Die kreisrunde Auslaßkammer 37 setzt sich unter Ausbildung des oben erwähnten Trillerpfeifenprofils in den Mundstückkanal 7 nach oben fort. Der stumpfwinklig zum Verbindungskanal 8 verlaufende Mundstückkanal 7 ist so ausgestaltet, daß die Spurgerade der einen Wand c tangential und diejenige der anderen Wand d etwa radial zum Zentrum Z'' der Wirbelkammer W gerichtet ist. Die Wand c hat auf halber Länge einen Knick im Sinne einer leichten Verengung nach außen hin.

Der Lufteinlaßkanal 9 ist so ausgerichtet, daß die Spurgerade der zur Kanalscheide 12 führenden Innenwand e und die der Außenwand f des Lufteinlaßkanals 9 sekantenförmig zur Dosiertrommel D ausgerichtet sind.

Durch den Lufteinlaßkanal 9 wird die Inhalationsluft von außen her durch Lufteinlaßschlitze 39 angesaugt. Die eine Hälfte der Lufteinlaßschlitze ist an der Gehäuseschale 32 und die andere Hälfte an der gegenüberliegenden Gehäuseschale 33 angebracht, so daß der Saugluftstrom vor dem Eintritt in den Lufteinlaßkanal 9 in zwei Teilströme aufgeteilt wird. Zu diesem Zweck wird im Träger 34 eine Spaltung des Lufteinlaßkanals 9 vorgenommen. Die Spaltung erfolgt durch einen Teilungszwickel 40, der die von beiden Breitseiten der Vorrichtung her angesaugten Teilströme zu einem Saugstrom vereinigt (s. Fig. 1 und Fig. 3 bis 6). Während die Auslaßkammer der Wirbelkammer W kreisrund ist, hat die Verweilzeitkammer 36 eine vom Kreisquerschnitt abweichende Form. Wie aus Fig. 3 ersichtlich, weist der dem oberen Ende des Verbindungskanals 8 zugewandte Wandabschnitt 36' eine geringere Wölbung und damit einen deutlich flacheren Verlauf auf, als der Hauptteil der Verweilzeitkammer 36. Relativ zu diesem abgeflachten Teil 36' ist der zentrale Durchlaß der Wirbelkannner W exzentrisch angeordnet.

Diese "Abflachung" führt überraschenderweise zu einem besseren Abtrag von evtl. abgelagertem pulverförmigen Arzneistoff.

Die deutliche Abflachung erstreckt sich nur über den oberen Halbkreis der Wirbelkammer W; die untere Hälfte, die durch das obere Ende des Verbindungskanals 8 unterbrochen ist, ist kreisrund, also konzentrisch zum dortigen Zentrum Z'' der Wirbelkammer W.

Um bei einer Fehlbedienung des Inhalators 1, also einem Blasen statt Saugen, den Austrag der Arzneistoff-Inhaliermenge auszuschließen, sind im Strömungsweg des Teilungszwickels 40 im Bereich des Lufteinlasses zwei Exhalations-Sperrzungen 41 angeordnet (s. Fig. 5). Sie wirken wie Rückschlagventile, wobei der innenseitige Rand der Lufteinlaßschlitze 39 die entsprechende Ventilsitzfläche bildet. Eine solche Exhalations-Sperrzunge 41 ist einfach aufgebaut und besteht aus einem Gummi- oder Kunststoffstreifenabschnitt entsprechender Flexibilität. Dieser wird einfach über den im Querschnitt dreieckigen Teilungszwickel 40 gelegt und in einem Halteschlitz 41 a zur Wand des Trägers 2 hin eingeklemmt. Der Keil des Teilungswickels 40 weist nach unten.

Die Funktion der Exhalations-Sperrzungen 41 ist auch ergonometrisch sichergestellt, da die Einlaßschlitze 39 nur bis etwa auf die Höhe des Zentrums Z'' der Wirbelkammer W reichen. Die Lufteinlaßschlitze 39 liegen somit im Koptbereich des Inhalators 1, während der Fußbereich desselben für das Greifen freiliegt. Insbesondere aus Fig. 1 geht auch eine schnabelartige Gestalt des besagten Kopfes des Inhalators 1 hervor. Das Mundstück M verjüngt sich zum freien Ende hin kontinuierlich, während am anderen Ende neben und im wesentlichen unterhalb der Wirbelkammer W eine Fingermulde 42 vorgesehen ist. Dadurch wird nicht nur die Benutzung des Inhalators 1 erleichtert, sondern auch die korrekte Handhabung desselben suggeriert. Beim Gebrauch des Inhalators liegt die Unterlippe des Benutzers an der Seitenfläche des Mundstücks oberhalb der Fingermulde 42 an.

Die besagte Fingermulde 42 ist derart gestaltet, daß bequem das Auflegen des Daumens ermöglicht wird.

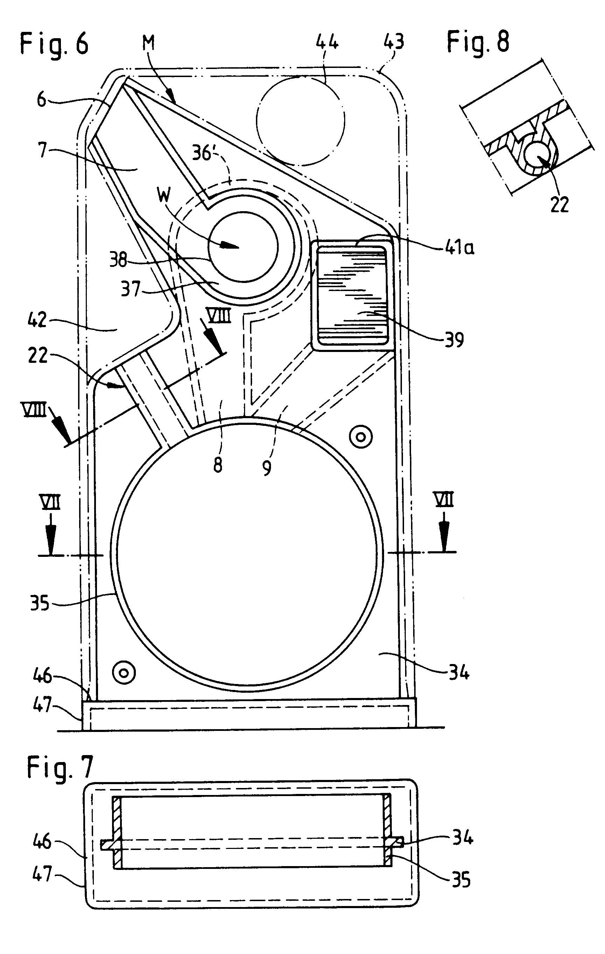

Eine den Inhalator 1 überfangende Schutzkappe 43 dient hygienischen Belangen (s. Fig. 6). Sie erhält im Deckenbereich ebenfalls eine Trockenmittelkammer 44, die in dem zwischen Schutzkappe 43 und Mundstück M verbleibenden Freiraum untergebracht wird. Bei der Schutzkappe 43 ist der Bereich des Drehknopfes 21 ausgespart (Ausnehmung 45).

Zur Anschlagbegrenzung der Schutzkappe 43 dient eine obere, ebenengleiche Schulter 46 eines am Träger 34 angeformten Standfußes 47. Die Schalenwände sind in diesem Bereich leicht ausgestellt, so daß der untere Rand der Schutzkappe 43 mittels einer Klemmhalterung unverlierbar gesichert ist.

Die Schutzkappe 43 überfangt auch die von den Schalen 32 und 33 gebildeten Breitseiten und Schmalseiten des Gehäuses 2 sowie dessen obere Stirnfläche.

An der dem Drehknopf 21 gegenüberliegenden Breitseite weist der Vorratsbehälter 3 einen durchsichtigen scheibenförmigen Abschnitt 48 als Sichtfenster auf (s. Fig. 2). Auf diese Weise kann der Pulverinhalt kontrolliert werden. Das besagte Sichtfenster fungiert zugleich als Fülldeckel. Dieser könnte auch als Träger für die statischen Auflockerungsfinger 29 dienen, wenn diese Version baulich zum Tragen käme. An der Innenseite des Fülldeckels könnte auch die beschriebene Trockenmittelkammer 30 sitzen.

Die Funktion des Inhalators 1 ist, kurz zusammengefaßt, wie folgt: Die über den Lufteinlaßkanal 9 angesaugte Luft strömt beim Inhalieren in die dosiert gefüllte Abteilkammer 11 und reißt den Arzneistoff 4 in den nach oben gerichteten Strömungsweg, d.h. in den Verbindungskanal 8. Dabei nimmt die Strömungsgeschwindigkeit bis in den Bereich der darüberliegenden Wirbelkammer W ab. Hier tritt der Luftstrom mit abnehmender Geschwindigkeit und entgegen der Schwerkraft der Teilchen in die Verweilkammer 36 und von dort quer übertretend durch den zentralen Durchlaß 38 unter weiterer Verlangsamung in die Auslaßkammer 37. Anschließend gelangt das Pulver/Luft-Gemisch in den Fußbereich des Mundstückkanals 7, der als Dispergierkammer wirkt. Aus dem daran anschließenden Diffusor, der durch das obere Ende des Mundstückkanals 7 gebildet wird, gelangt das Medikament über die Austrittsöffnung 6 an den Zielort (Bronchien, Lunge). Der Strömungsweg im Dispergierteil I gleicht zwei hintereinander gezogenen Schleifen gleichen Drehsinns.

Durch die kontinuierliche Verringerung der Strömungsgeschwindigkeit bis hin zur Austrittsöffnung 6 am Mundstückkanal 7 wird die Wahrscheinlichkeit der Prallabscheidung relativ kleiner Partikel im Rachenraum stark vermindert.

## Patentansprüche

1. Vorrichtung zum Inhalieren eines pulverförmigen, pharmakologisch wirksamen Arzneistoffs, bestehend aus einem Vorratsbehälter (3) für den Arzneistoff (4), einer handbetätigten Dosiereinrichtung zur Bereitstellung einer abgeteilten Inhalierstoffmenge, die von dem beim Inhalieren angesaugten Luftstrom erfaßt und verwirbelt wird, einem Lufteinlaßkanal (9), einer Wirbelkammer (W) und einem Verbindungskanal (8) zwischen der Wirbelkammer (W) und der Dosiereinrichtung sowie einem von der Wirbelkammer (W) ausgehenden Mundstückkanal (7), dadurch gekennzeichnet, daß die Dosiereinrichtung aus einer Dosiertrommel besteht, bei der der Vorratsbehälter (3) für den Arzneistoff im Innenraum (5) der Dosiertrommel D angeordnet ist, und daß der Lufteinlaßkanal (9) und der Verbindungskanal (8) V-förmig aufeinander und eine durch die Dosiertrommel D im Scheitel der beiden Luftkanäle (8) und (9) bereitgestellte, in Form einer Kuppel vorliegende, abgeteilte Arzneistoffmenge zulaufen.

2. Vorrichtung nach Anspruch 1, dadurch gekennzeichnet, daß der Verbindungskanal (8) radial zur Dosiertrommel (D) verläuft.

3. Vorrichtung nach einem oder mehreren der vorhergehenden Ansprüche, dadurch gekennzeichnet, daß der Verbindungskanal (8) sich zur Wirbelkammer (W) hin verbreitert und die Spurgerade der einen Wand (a) im wesentlichen radial zum Zentrum (Z'') der Wirbelkammer (W) liegt und diejenige der anderen Wand (b) im wesentlichen tangential dazu verläuft.

4. Vorrichtung nach einem oder mehreren der vorhergehenden Ansprüche, dadurch gekennzeichnet, daß der Mundstückkanal (7) stumpfwinklig zum Verbindungskanal (8) verläuft und die Spurgerade der einen Wand (c) etwa tangential und diejenige der anderen Wand (d) etwa radial zum Zentrum (Z'') der Wirkelkammer (W) gerichtet sind.

5. Vorrichtung nach einem oder mehreren der vorhergehenden Ansprüche, dadurch gekennzeichnet, daß die Spurgeraden der Innenwand (e) und Außenwand (f) des Lufteinlaßkanals (9) sekantenförmig zur Dosiertrommel (D) gerichtet sind.

6. Vorrichtung nach einem oder mehreren der vorhergehenden Ansprüche, dadurch gekennzeichnet, daß die Dosiertrommel (D) eine mit Abteildurchbrüchen (14) ausgestattete Drehhülse (15) besitzt.

7. Vorrichtung nach einem oder mehreren der vorhergehenden Ansprüche, dadurch gekennzeichnet, daß die Drehhülse (15) von der Mantelwand eines Topfes gebildet ist, an dessen Boden (18) ein Drehknopf (21) angeordnet ist.

8. Vorrichtung nach einem oder mehreren der vorhergehenden Ansprüche, gekennzeichnet durch ein den Drehsinn bestimmendes Rastgesperre (22) zwischen Drehhülse (15) und Gehäuse (2) der Vorrichtung.

9. Vorrichtung nach einem oder mehreren der vorhergehenden Ansprüche, dadurch gekennzeichnet, daß die Drehhülse (15) von einer Ringwand (16) umfaßt ist und über einen Teilwinkelbereich von einem inneren Wandabschnitt (17) unterfangen ist, welcher im Bereich des V-Scheitels (10) den Boden der von jeweils einem der Durchbrüche (14) gebildeten Abteilkammer (11) bildet.

10. Vorrichtung nach einem oder mehreren der vorhergehenden Ansprüche, dadurch gekennzeichnet, daß die Querwände (13) der Durchbrüche (14) keilförmig in Richtung zum Zentrum (Z') der Dosiertrommel (D) hin verlaufen.

11. Vorrichtung nach einem oder mehreren der vorhergehenden Ansprüche, dadurch gekennzeichnet, daß ausgehend vom Boden (18) des Topfes und/oder dem Gehäuse (2) Auflockerungsfinger (29) in die Dosiertrommel (D) vorstehen.

12. Vorrichtung nach einem oder mehreren der vorhergehenden Ansprüche, dadurch gekennzeichnet, daß dem Boden (20) des Topfes oder der Innenseite eines Gehäuse-Fülldeckels eine Trockenmittelkammer (30) zugeordnet ist.

13. Vorrichtung nach einem oder mehreren der vorhergehenden Ansprüche, dadurch gekennzeichnet, daß am Einsaugende des Lufteinlaßkanals (9) quergerichtete Lufteinlaßschlitze (39) angeordnet sind.

14. Vorrichtung nach einem oder mehreren der vorhergehenden Ansprüche, dadurch gekennzeichnet, daß die Lufteinlaßschlitze (39) auf beiden Breitseiten des Vorrichtungs-Gehäuses (2) vorgesehen sind.

15. Vorrichtung nach einem oder mehreren der vorhergehenden Ansprüche, gekennzeichnet durch einen Teilungszwickel (40) im Strömungsweg des Lufteinlaßkanals (9).

16. Vorrichtung nach einem oder mehreren der vorhergehenden Ansprüche, dadurch gekennzeichnet, daß den Lufteinlaßschlitzen (39) Exhalations-Sperrzungen (41) zugeordnet sind.

17. Vorrichtung nach einem oder mehreren der vorhergehenden Ansprüche, dadurch gekennzeichnet, daß die Lufteinlaßschlitze (39) im Kopfbereich des Inhalators angeordnet sind und etwa bis auf die Höhe des Zentrums (Z'') der Wirbelkannner (W) reichen.

18. Vorrichtung nach einem oder mehreren der vorhergehenden Ansprüche, dadurch gekennzeichnet, daß unterhalb der Wirbelkammer (W) eine Fingermulde (42) am Inhalator-Gehäuse (2) angeordnet ist.

19. Vorrichtung nach einem oder mehreren der vorhergehenden Ansprüche, gekennzeichnet durch eine das Gehäuse (2) überfangende Schutzkappe (43).

20. Vorrichtung nach einem oder mehreren der vorhergehenden Ansprüche, gekennzeichnet durch eine der Schutzkappe (43) zugeordnete Trockenmittelkammer (44).

21. Vorrichtung nach einem oder mehreren der vorhergehenden Ansprüche, dadurch gekennzeichnet, daß die Kanäle (7, 8, 9) und die Wirbelkammer (W) in einem von zwei Gehäuseschalen (32, 33) umschließbaren Träger (34) untergebracht sind, in dem auch die Dosiertrommel (D) gelagert ist, wobei die Gehäuseschalen (32, 33) die Abdeckung für die Kanäle (7, 8, 9) und die Wirbelkammer (W) bilden.

22. Vorrichtung nach einem oder mehreren der vorhergehenden Ansprüche, dadurch gekennzeichnet, daß die Wirbelkammer (W) durch eine Verweilzeitkammer (36) und durch eine Austragskammer (37) gebildet wird, die über einen zentralen Durchlaß (38) miteinander verbunden sind, und daß sich an die Austragskammer (37) ein als Dispergierkammer wirkender Mundstückkanal (7) anschließt.

23. Vorrichtung nach einem oder mehreren der vorhergehenden Ansprüche, dadurch gekennzeichnet, daß der Träger (34) einen Standfiß (47) aufweist, dessen Schulter (46) einen klemmgesicherten Aufsteckbegrenzungsanschlag für die Schutzkappe (43) bildet.

24. Vorrichtung nach einem oder mehreren der vorhergehenden Ansprüche, dadurch gekennzeichnet, daß der dem oberen Ende des Verbindungskanals (8) zugewandte Wandabschnitt (36') der Verweilzeitkammer (36) eine geringere Wölbung besitzt als der restliche Teil der Verweilzeitkammer (36) und damit an der Übergangsstelle in die Verweilzeitkammer (36) abgeflacht ist, und daß der zentrale Durchlaß (38) in der Verweilzeitkammer (36) relativ zu dem abgeflachten Wandungsabschnitt (36') exzentrisch angeordnet ist.

## Claims

1. Device for inhalation of a powdery, pharmacologically active drug, consisting of a supply container (3) for the drug (4), a manually actuated metering unit for providing a portioned-off quantity of inhalation substance which is picked up by the air current drawn in during inhalation and is swirled, an air inlet channel (9), a swirl chamber (W), and a connection channel (8) between the swirl chamber (W) and the metering unit, as well as a mouthpiece channel (7) issuing from the swirl chamber (W), characterized in that the metering unit consists of a metering drum, the supply container (3) for the drug being arranged in the interior (5) of the metering drum D, and that the air inlet channel (9) and the connection channel (8) run towards each other in a V-shape and towards a portioned-off quantity of drug present in the shape of a dome and provided by the metering drum D at the apex of the two air channels (8) and (9).

2. Device according to Claim 1, characterized in that the connection channel (8) runs radially with respect to the metering drum (D).

3. Device according to one or more of the preceding claims, characterized in that the connection channel (8) widens towards the swirl chamber (W), and the trace line of the one wall (a) lies essentially radially with respect to the centre (Z'') of the swirl chamber (W) and that of the other wall (b) runs essentially tangentially thereto.

4. Device according to one or more of the preceding claims, characterized in that the mouthpiece channel (7) runs at an obtuse angle with resect to the connection channel (8), and the trace line of one wall (c) is directed approximately tangentially, and that of the other wall (d) approximately radially, with respect to the centre (Z'') of the swirl chamber (W).

5. Device according to one or more of the preceding claims, characterized in that the trace lines of the inner wall (e) and outer wall (f) of the air inlet channel (9) are directed in secant formation with respect to the metering drum (D).

6. Device according to one or more of the preceding claims, characterized in that the metering drum (D) has a rotary sleeve (15) equipped with portion apertures (14).

7. Device according to one or more of the preceding claims, characterized in that the rotary sleeve (15) is formed by the circumferential wall of a pot, on the base (18) of which a turning knob (21) is arranged.

8. Device according to one or more of the preceding claims, characterized by a catch mechanism (22), determining the direction of rotation, between rotary sleeve (15) and housing (2) of the device.

9. Device according to one or more of the preceding claims, characterized in that the rotary sleeve (15) is surrounded by an annular wall (16) and is underpinned, over a partial angle range, by an inner wall section (17) which, in the area of the V apex (10), forms the base of the portion chamber (11) formed by in each case one of the apertures (14).

10. Device according to one or more of the preceding claims, characterized in that the transverse walls (13) of the apertures (14) run in a wedge formation in the direction of the centre (Z') of the metering drum (D).

11. Device according to one or more of the preceding claims, characterized in that, issuing from the base (18) of the pot and/or from the housing (2), loosening fingers (29) protrude into the metering drum (D).

12. Device according to one or more of the preceding claims, characterized in that a drying agent chamber (30) is allocated to the base (20) of the pot or to the inner side of a housing-filling lid.

13. Device according to one or more of the preceding claims, characterized in that transversely directed air inlet slots (39) are arranged at the intake end of the air inlet channel (9).

14. Device according to one or more of the preceding claims, characterized in that the air inlet slots (39) are provided on both broad sides of the device housing (2).

15. Device according to one or more of the preceding claims, characterized by a dividing gusset (40) in the flow path of the air inlet channel (9).

16. Device according to one or more of the preceding claims, characterized in that the air inlet slots (39) are allocated exhalation barrier tongues (41).

17. Device according to one or more of the preceding claims, characterized in that the air inlet slots (39) are arranged in the head area of the inhaler and reach to about the height of the centre (Z'') of the swirl chamber (W).

18. Device according to one or more of the preceding claims, characterized in that a finger depression (42) is arranged on the inhaler housing (2) below the swirl chamber (W).

19. Device according to one or more of the preceding claims, characterized by a protective cap (43) engaging over the housing (2).

20. Device according to one or more of the preceding claims, characterized by a drying agent chamber (44) allocated to the protective cap (43).

21. Device according to one or more of the preceding claims, characterized in that the channels (7, 8, 9) and the swirl chamber (W) are accommodated in a support structure (34) which can be enclosed by two housing shells (32, 33) and in which the metering drum (D) is also mounted, the housing shells (32, 33) forming the covering for the channels (7, 8, 9) and the swirl chamber (W).

22. Device according to one or more of the preceding claims, characterized in that the swirl chamber (W) is formed by a dwell-time chamber (36) and by a discharge chamber (37) which are connected to each other via a central passage (38), and in that a mouthpiece channel (7) acting as dispersing chamber adjoins the discharge chamber (37).

23. Device according to one or more of the preceding claims, characterized in that the support structure (34) has a stand (47), the shoulder (46) of which forms a push-on limit stop on which the protective cap (43) can be securely clamped.

24. Device according to one or more of the preceding claims, characterized in that the wall section (36') of the dwell-time chamber (36) directed towards the upper end of the connection channel (8) has a smaller curvature than the remaining part of the dwell-time chamber (36) and is consequently flattened at the point of transfer into the dwell-time chamber (36), and in that the central passage (38) in the dwell-time chamber (36) is arranged eccentrically relative to the flattened wall section (36').

## Revendications

1. Dispositif servant à l'inhalation d'un médicament pulvérulent, actif sur le plan pharmacologique, constitué d'un récipient de stockage (3) pour le médicament (4), d'un dispositif de dosage actionné à la main pour la mise à disposition d'une quantité dosée à inhaler, qui est emportée et mise en tourbillon par le flux d'air aspiré, d'un canal d'entrée d'air (9), d'une chambre de tourbillonnement (W) et d'un canal de liaison (8) entre la chambre de tourbillonnement (W) et le dispositif de dosage, ainsi que d'un canal d'embouchure (7) partant de la chambre de tourbillonnement (W), caractérisé en ce que le dispositif de dosage est constitué d'un tambour de dosage, pour lequel le récipient de stockage (3) pour le médicament est disposé dans l'espace intérieur (5) du tambour de dosage (D), et en ce que le canal d'entrée d'air (9) et le canal de liaison (8) concourent en forme de V l'un vers l'autre et vers une quantité dosée de médicament, présente sous forme d'une coupole, mise à disposition par le tambour de dosage (D) au sommet des deux canaux d'air (8) et (9).

2. Dispositif suivant la revendication 1, caractérisé en ce que le canal de liaison (8) s'étend de manière radiale par rapport au tambour de dosage (D).

3. Dispositif suivant l'une ou plusieurs des revendications précédentes, caractérisé en ce que le canal de liaison (8) s'élargit vers la chambre de tourbillonnement (W) et la ligne droite d'une paroi (a) se trouve essentiellement en position radiale par rapport au centre (Z'') de la chambre de tourbillonnement (W) et celle de l'autre paroi (b) s'étend essentiellement tangentiellement à celle-ci.

4. Dispositif suivant l'une ou plusieurs des revendications précédentes, caractérisé en ce que le canal d'embouchure (7) fait un angle obtus avec le canal de liaison (8) et que la ligne droite d'une paroi (c) est orientée de manière environ tangentielle et celle de l'autre paroi (d) de manière environ radiale par rapport au centre (Z'') de la chambre de tourbillonnement (W).

5. Dispositif suivant l'une ou plusieurs des revendications précédentes, caractérisé en ce que les lignes droites de la paroi intérieure (e) et de la paroi extérieure (f) du canal d'entrée d'air (9) sont orientées de manière sécante par rapport au tambour de dosage (D).

6. Dispositif suivant l'une ou plusieurs des revendications précédentes, caractérisé en ce que le tambour de dosage (D) possède une enveloppe tournante (15) équipée de passages compartimentés (14).

7. Dispositif suivant l'une ou plusieurs des revendications précédentes, caractérisé en ce que l'enveloppe tournante (15) est constituée par l'enveloppe d'un pot, sur le fond (18) duquel est disposé bouton rotatif (21).

8. Dispositif suivant l'une ou plusieurs des revendications précédentes, caractérisé par un blocage de verrouillage (22) définissant le sens de rotation situé entre l'enveloppe tournante (15) et le boîtier (2).

9. Dispositif suivant l'une ou plusieurs des revendications précédentes, caractérisé en ce que l'enveloppe tournante (15) est entourée d'une paroi annulaire (16) et est soutenue sur une partie angulaire par une partie intérieure de paroi (17), laquelle forme à proximité du sommet en V (10) le fond du compartiment (11) formé respectivement par un des passages (14).

10. Dispositif suivant l'une ou plusieurs des revendications précédentes, caractérisé en ce que les parois transversales (13) des passages (14) s'étendent en forme de coin vers le centre (Z') du tambour de dosage (D).

11. Dispositif suivant l'une ou plusieurs des revendications précédentes, caractérisé en ce que, partant du fond (18) du pot et/ou du boîtier (2), des doigts de mélange (29) dépassent dans le tambour de dosage (D).

12. Dispositif suivant l'une ou plusieurs des revendications précédentes, caractérisé en ce qu'une chambre pour agent de déshydratation (30) est prévue au fond du pot ou du côté intérieur d'un couvercle de remplissage du boitier.

13. Dispositif suivant l'une ou plusieurs des revendications précédentes, caractérisé en ce que des fentes d'entrée d'air (39) orientées transversalement sont disposées à l'extrémité d'aspiration du canal d'entrée d'air (9).

14. Dispositif suivant l'une ou plusieurs des revendications précédentes, caractérisé en ce que les fentes d'entrée d'air (39) sont prévues sur les deux grands côtés du boîtier (2) du dispositif.

15. Dispositif suivant l'une ou plusieurs des revendications précédentes, caractérisé par un coin de séparation (40) dans le chemin d'écoulement du canal d'entrée d'air (9).

16. Dispositif suivant l'une ou plusieurs des revendications précédentes, caractérisé en ce que des languettes de blocage d'exhalation (41) correspondent aux fentes d'entrée d'air (39).

17. Dispositif suivant l'une ou plusieurs des revendications précédentes, caractérisé en ce que les fentes d'entrée d'air (39) sont disposées dans la zone de tête de l'inhalateur et arrivent environ jusqu'à la hauteur du centre (Z'') de la chambre de tourbillonnement (W).

18. Dispositif suivant l'une ou plusieurs des revendications précédentes, caractérisé en ce que, en dessous de la chambre de tourbillonnement (W), un renfoncement pour les doigts (42) est disposé dans le boîter (2) de l'inhalateur.

19. Dispositif suivant l'une ou plusieurs des revendications précédentes, caractérisé par un capot de protection (43) recouvrant le boitier (2).

20. Dispositif suivant l'une ou plusieurs des revendications précédentes, caractérisé par une chambre pour agent de déshydratation (44) correspondant au capot de protection (43).

21. Dispositif suivant l'une ou plusieurs des revendications précédentes, caractérisé en ce que les canaux (7, 8, 9) et la chambre de tourbillonnement (W) sont abrités dans un support (34) pouvant être entouré par deux coquilles de boîtier (32, 33), dans lequel le tambour de dosage (D) est également supporté, les coquilles de boîtier (32, 33) formant le recouvrement pour les canaux (7, 8, 9) et la chambre de tourbillonnement (W).

22. Dispositif suivant l'une ou plusieurs des revendications précédentes, caractérisé en ce que la chambre de tourbillonnement (W) est formée par une chambre de séjour (36) et une chambre de sortie (37), qui sont reliées entre elles par un passage central (38) et en ce qu'un canal d'embouchure (7) agissant comme chambre de dispersion se raccorde à la chambre de sortie (37).

23. Dispositif suivant l'une ou plusieurs des revendications précédentes, caractérisé en ce que le support (34) présente un pied fixe (47), dont l'épaulement (46) forme une butée de limite d'enfichage fixée par pinçage pour le capot de protection (43).

24. Dispositif suivant l'une ou plusieurs des revendications précédentes, caractérisé en ce que la partie de paroi (36') faisant face à la partie supérieure du canal de liaison (8) présente une courbure plus faible que le reste de la chambre de séjour (36) et est donc aplatie au point de transfert vers la chambre de séjour (36) et en ce que le passage central (38) dans la chambre de séjour (36) est disposé de manière excentrique par rapport à la partie de paroi aplatie (36').
